# EUROPEAN PATENT SPECIFICATION

(11) **EP 2 510 115 B1**
(45) Date of publication and mention of the grant of the patent: **13.08.2014**
(21) Application number: 10790934.3
(22) Date of filing: 09.12.2010
(51) Int. Cl.: C12Q 1/68

(54) **SINGLE NUCLEOTIDE POLYMORPHISMS ASSOCIATED WITH DIETARY WEIGHT LOSS**
Mit Gewichtsverlust infolge einer Diät assoziierte einzelne Nukleotid-Polymorphismen
Polymorphismes de nucléotides uniques liés à la perte de poids alimentaire

(30) Priority: 09.12.2009 EP 09178544
(43) Date of publication of application: 17.10.2012
(73) Proprietor: DSM IP Assets B.V., 6411 TE Heerlen (NL)
(72) Inventor: SANCAK, Özgür, 4303 Kaiseraugst (CH)
(74) Representative: Schwander, Kuno
(86) International application number: PCT/EP2010/069288
(87) International publication number: WO 2011/070115

(56) References cited:
- WO-A1-2009/077614
- US-A1- 2006 252 050
- TENG ALLEN C T ET AL: "Functional characterization of a promoter polymorphism that drives ACSL5 gene expression in skeletal muscle and associates with diet-induced weight loss.", THE FASEB JOURNAL : OFFICIAL PUBLICATION OF THE FEDERATION OF AMERICAN SOCIETIES FOR EXPERIMENTAL BIOLOGY, vol. 23, no. 6, June 2009 (2009-06), pages 1705-1709, XP002572847, ISSN: 1530-6860
- SEIP RICHARD L ET AL: "Physiogenomic comparison of human fat loss in response to diets restrictive of carbohydrate or fat", NUTRITION & METABOLISM, vol. 5, no. 1, 6 February 2008 (2008-02-06), page 4, XP021038425, BIOMED CENTRAL. LONDON, GB ISSN: 1743-7075
- DOLORES CORELLA ET AL: "APOA5 gene variation modulates the effects of dietary fat intake on body mass index and obesity risk in the Framingham Heart Study", JOURNAL OF MOLECULAR MEDICINE, vol. 85, no. 2, 9 January 2007 (2007-01-09), pages 119-128, XP019467095, SPRINGER, BERLIN, DE ISSN: 1432-1440
- ORDOVAS J M ET AL: "GENETIC VARIATION AND LIPID METABOLISM: MODULATION BY DIETARY FACTORS", CURRENT CARDIOLOGY REPORTS, vol. 7, no. 6, 1 January 2005 (2005-01-01) , pages 480-486, XP009076538, CURRENT SCIENCE, PHILADELPHIA, PA, US ISSN: 1523-3782
- VINCENT S ET AL: "GENETIC POLYMORPHISMS AND LIPOPROTEIN RESPONSES TO DIETS", PROCEEDINGS OF THE NUTRITION SOCIETY, LONDON, GB, vol. 61, no. 4, 1 November 2002 (2002-11-01), pages 427-434, XP009034584, ISSN: 0029-6651
- PEARSON THOMAS A ET AL: "How to interpret a genome-wide association study.", JAMA : THE JOURNAL OF THE AMERICAN MEDICAL ASSOCIATION, vol. 299, no. 11, 19 March 2008 (2008-03-19), pages 1335-1344, XP002623925, ISSN: 1538-3598 -& PEARSON THOMAS A ET AL: "Corrections: How to interpret a genome-wide association study.", JAMA : THE JOURNAL OF THE AMERICAN MEDICAL ASSOCIATION, vol. 299, no. 18, 14 May 2008 (2008-05-14) , page 2150, XP002627217,

## Description

### Field of the invention

The present invention is in the field of obesity. More in particular it relates to a genetic polymorphism and its effect on dietary weight loss intervention programs. Moreover, the present invention pertains to genetic tests and methods using the polymorphisms, particularly methods to predict an obese individual's likelihood to complete a dietary weight loss intervention program successfully.

### Background of the invention

Obesity is a worldwide epidemic found across all age groups. Especially in industrialized countries, it has increased at a fast rate over the past two decades and is now a worldwide leading public health problem. For example, while in 1996 26 % adult Americans were overweight and 10 % severely so, currently more than 65 % are overweight, with nearly 31 % meeting the criteria for obesity. As obesity portends an epidemic of related chronic diseases such as type-2 diabetes, hypertension and cardiovascular events, people with obesity especially people with extreme obesity are at risk for many health problems. The economic cost attributable to obesity in the United States alone has been estimated to be as high as $100 billion per year and includes not only direct health care costs but also the cost of lost productivity in affected individuals.

While diet and lifestyle contribute to obesity and the trend of decreased physical activity and increased caloric intake is probably responsible for the recent rise in obesity, it is important to understand that genetics plays a key role. Each individual's genetic background remains an important determinant of susceptibility to obesity. For instance, half of the population variation in body mass index (BMI), a common measure of obesity, is determined by inherited factors.

Many studies have reported that common genetic variants, usually single nucleotide polymorphisms (SNPs), are associated with an increased risk for obesity.

Two approaches have been used to date to find these variants, linkage analysis and association studies. Although on the basis of linkage studies some regions have been repeatedly implicated to play a role in obesity, no genes have been found in these regions that have been seen to contribute to the disease. By using association studies several associations between obesity or obesity-related traits and common genetic variants have been reported. Unfortunately, many of the reported associations have not been consistently replicated. In WO2009077614, SNPs are used for assessing whether an individual will benefit from a low fat/high carbohydrate diet or a high fat/low carbohydrate diet.

Altering dietary habits is the cornerstone of weight loss intervention programs for overweight and obese patients. As it is unlikely that all overweight or obese individuals can lose weight with a standard protocol, dietary guidance should be individualized to allow for personalized approaches and recommendations and to increase success rates in these programs. Despite the increasing knowledge of loci and genes associated with obesity and obesity-related traits, no useful genetic variants exist on the basis of which dietary weight loss intervention programs can be tailored for overweight or obese individuals. A recent study even drew the conclusion that common SNPs in a panel of obesity-related candidate genes play a minor role, if any, in modulating weight changes induced by certain diets (see Sorensen et al., 2006). Given that no predictive genetic information about the response to diet is available and dietary treatment of obesity could be dramatically improved if predictive genetic information about the genetic response to diet was available, there is a clear need in the art for identifying genetic variants that predict the response of an overweight or obese individual to a dietary weight loss intervention program, for instance SNPs that predict the likelihood that an overweight or obese individual successfully completes such a program. The present invention meets these needs.

### Summary of the invention

It was found in accordance with the present invention that a markers exists that is associated with weight loss. The markers can be used to predict the likelihood that an individual, such as an overweight or obese individual, is successful in a dietary weight loss intervention program. Successful in this respect *inter alia* means that the individual successfully completes the dietary weight loss intervention program, *e.g*. the individual loses the target amount of weight and/or fat mass. Loss of a target amount of weight and/or fat mass can be accomplished by following *e.g*. a hypo-caloric diet. So, successful completion can be the consequence of the choice of diet. An individual may benefit more from one diet compared to another diet (*e.g*. a high fat/low carbohydrate hypo-caloric diet compared to a low fat/high carbohydrate hypo-caloric diet or *vice versa).* The markers provided herein can also be used to determine the optimal diet for an individual. The term "associated with" in connection with the relationship between a genetic characteristic, *e.g*., a marker, allelic variant, haplotype, or polymorphism, and a trait means that there is a statistically significant level of relatedness between them based on any generally accepted statistical measure of relatedness. Those skilled in the art are familiar with selecting an appropriate statistical measure for a particular experimental situation or data set. Examples of suitable statistical methods are described herein. Accordingly, the present invention is directed to methods wherein use is made of the genetic characteristics in predicting likely response, preferably likely successful response, to weight loss and weight management. The invention also provides kits for use in the methods and uses of the present invention, *e.g*. kits to determine whether an individual is likely to successfully complete a specific diet on the basis of analysis of genetic markers *e.g.* SNPs. The markers can for instance be found in genes associated with overweight, obesity or obesity-related metabolic traits. The resulting information can be used to classify individuals such as overweight or obese individuals based on their genetic tendency to have success with certain types of diet. This will help professionals in the field of weight management to improve targeting these individuals with appropriate (nutritional) advice regarding their weight management. As a result thereof, the success rate of dietary weight loss intervention programs will increase.

### Detailed description of the invention

The invention is based on the finding that a single nucleotide polymorphisms (SNP), rs1900075 is associated with weight loss. rs1900075 can be used to predict the likelihood of success of an individual, preferably an overweight or obese individual, in a dietary weight loss intervention program. If the SNP marker rs1900075 is identified, the likelihood of success of an individual, preferably an overweight or obese individual, in a dietary weight loss intervention program is higher than when the SNP is not identified. Preferably, the program comprises administration of a hypo-caloric diet. If an individual has the SNP rs1900075, the individual will lose more weight during a dietary weight loss intervention program than an individual who does not have the SNP rs1900075. The SNP rs1900075 can thus be used to determine which individual is likely to lose more weight and which individual is likely to lose less weight (and therefore needs *e.g.* more coaching) in a dietary weight loss intervention program, *e.g*. a dietary weight loss intervention program comprising administration of a hypo-caloric diet.

Furthermore, the invention provides a SNP which can be used to determine the type of diet an individual, preferably an overweight or obese individual, is most likely to complete successfully. The SNP rs1900075 can be used to determine whether an individual will benefit more from a high fat/low carbohydrate diet or from a low fat/high carbohydrate diet (the SNPs thus also can be used to determine whether an individual will benefit more from a dietary weight loss intervention program comprising a high fat/low carbohydrate diet or from a dietary weight loss intervention program comprising a low fat/high carbohydrate diet). The SNP rs1900075 can be used to determine which individual is likely to lose more weight on a high fat/low carbohydrate diet than on a low fat/high carbohydrate diet and which individual is likely to lose more weight on a low fat/high carbohydrate diet than on a high fat/low carbohydrate diet. In other words, the SNP rs1900075 can be used to determine whether an individual is likely to lose more weight on a high fat/low carbohydrate diet than on a low fat/high carbohydrate diet or whether an individual is likely to lose more weight on a low fat/high carbohydrate diet than on a high fat/low carbohydrate diet. An individual having a SNP rs1900075 is likely to lose more weight on a high fat/low carbohydrate diet than on a low fat/high carbohydrate diet. An individual having a SNP rs1900075 is more likely to complete a high fat/low carbohydrate diet than a low fat/high carbohydrate diet successfully. Again, the diet may be part of a dietary weight loss intervention program. On the basis of the SNP identified in the present invention individuals can be classified into individuals that have a higher tendency to successfully complete dietary weight loss intervention programs comprising low fat/high carbohydrate diets than dietary weight loss intervention programs comprising high fat/low carbohydrate diets, and into individuals that have a higher tendency to successfully complete dietary weight loss intervention programs comprising high fat/low carbohydrate diets than dietary weight loss intervention programs comprising low fat/high carbohydrate diets.

The invention provides a polymorphism that is useful in predicting the out-come of weight loss intervention programs, particularly programs having a component of dietary intervention *e.g.* diets. The present invention is directed to methods capable of predicting likely response, preferably likely successful response, to weight loss and weight management based on genetic polymorphisms and methods to assess an individual's likelihood of responsiveness to weight management programs by genetically classifying individuals as likely susceptible or likely resistant to weight management programs, *e.g*. weight management programs comprising a dietary intervention. The results as found herein indicate that individuals carrying certain polymorphisms have great difficulty in managing their weight and further shows that the polymorphisms can predict the outcome of body weight reduction strategies that are based on dietary intervention such as diets. Consequently, the identification of the polymorphisms can help weight management professionals to design alternative weight management programs for these individuals.

One or more of the polymorphisms may be part of a haplotype which may have an association link with the likelihood of an individual to successfully or unsuccessfully complete a certain dietary weight loss intervention program. As used herein, "haplotype" refers to a set of alleles found at linked polymorphic sites on a single chromosome. The linked sites may include part of a gene, an entire gene, several genes, or a region devoid of genes (but which perhaps contains a DNA sequence that regulates the function of nearby genes). The haplotype preserves information about the phase of the polymorphic nucleotides, that is, which set of variances were inherited from one parent (and are therefore on one chromosome) and which from the other. In a preferred embodiment the programs comprise dietary intervention either alone or as a major component. Next to suitable diets, *i.e.* personalized diets based on the genetic profile of an individual, weight loss intervention programs may however also include other components such as *e.g.* drug treatment, surgical treatment *e.g.* liposuction, behavioural therapy, increase in physical activity and dietary supplement treatment.

As on the basis of the genetic markers according to the present invention, in particular the genetic marker rs1900075, individuals such as over-weight or obese individuals may be identified that have an increased likelihood to successfully complete a dietary weight loss intervention program comprising a low fat/high carbohydrate diet compared to a dietary weight loss intervention program comprising a high fat/low carbohydrate diet and *vice versa.* The identification of SNPs in an individual that are associated with specific types of diets can help weight management professionals to design suitable dietary weight loss intervention programs for these individuals.

In an aspect the invention relates to the use of a single nucleotide polymorphism (SNP) consisting of rs1900075 for predicting the likelihood of success of an individual in a dietary weight loss intervention program comprising subjecting the individual to a hypo-caloric diet.

The individual may be overweight or obese. The invention also relates to the use of a single nucleotide polymorphism (SNP) rs1900075 for determining a diet an individual is most likely to complete successfully. The diet may be a high fat/low carbohydrate or a low fat/high carbohydrate diet. The diet may be a hypo-caloric diet.

In an aspect the invention relates to the use of a genetic marker such as a polymorphism, *e.g.* a SNP, rs1900075 for predicting the likelihood of success of an individual in a dietary weight loss intervention program comprising subjecting the individual to a hypo-caloric diet. In other words, the data provided herein show that a correlation, association, linkage or other relation between a specific marker and the likelihood of success in a dietary weight loss intervention program can be established.

In a further aspect the invention relates to the use of a genetic marker such as a polymorphism, *e.g.* a SNP, rs1900075 for determining a diet an individual is most likely to benefit from in a dietary weight loss intervention program. The dietary weight loss intervention programs comprise treatment of an individual with a diet, *e.g.* a hypo-caloric diet. Diets used in dietary weight loss intervention programs designed to treat individuals are well known to the skilled person. These include, but are not limited to, low energy/low calorie diets. Preferred diets in the light of the present invention include, but are not limited to, high fat/low carbohydrate diets or low fat/high carbohydrate diets. The high fat/low carbohydrate or low fat/high carbohydrate diets may be hypo-energetic diets (hypo-caloric diets). In an embodiment the individual is overweight or obese. An "individual" as used in the present application refers to a human.

An "overweight individual", as used herein, refers to an individual fulfilling the normal definition of overweight individual as defined by the medical knowledge at the time of diagnosis. Useful criteria for defining an individual as overweight include, but are not limited to, a body mass index (BMI) of 25-29.9, male individual with a waist measurement greater than 40 inches (102 cm), female individual with a waist measurement greater than 35 inches (88 cm), and all individuals with a waist-to-hip ratio of 1.0 or higher. An "obese individual", as used herein, refers to an individual fulfilling the normal definition of obese individuals as defined by the medical knowledge at the time of diagnosis. Useful criteria for defining an individual as obese include, but are not limited to, a body mass index (BMI) of 30 or higher. The definitions for overweight or obese can vary in children or teenagers. The definitions are definitions at the time of observation of the individual in the light of the then current medical knowledge. The definitions may thus change.

A "hypo-energetic (hypo-caloric) diet" as used herein means a diet wherein the daily energy intake is less than the daily energy requirement, *e.g.* a diet with an energy deficiency of at least 100, 200, 300, 400, 600, 800, 1000, 1200, 1500 or 2000 kcal/day. "High fat" diets as used herein means diets having at least 30 %, preferably at least 40 %, more preferably 40 to 45 % of energy from fat. "Low fat" diets as used herein means diets having less than 30 %, preferably less than 25 %, more preferably 20 to 25 % of energy from fat. "Low carbohydrate" diets as used herein means diets having less than 50 %, preferably less than 45 %, more preferably 40 to 45 % of energy from carbohydrate. "High carbohydrate" diets as used herein means diets having at least 50 %, preferably at least 60 %, more preferably 60 to 65 % of energy from carbohydrate. The diets may further contain other components such as *e.g.* proteins. The diets may have *e.g.* 15 % of energy from proteins. Preferably, the individuals on the dietary intervention program do not consume alcohol. Where exclusion of alcohol is not possible, intake should be minimal, with an upper limit of two glasses (2 x 150 ml) in total. Energy from alcohol should be subtracted from total energy intake and thereafter macronutrient intake should be calculated on the remaining energy. Where possible, viscous soluble fibres should be avoided in the diets, since they are thought to have the greatest impact on glucose and lipid metabolism (*e.g.* oats and guar gum).

Furthermore, it may be attempted to standardise other sources of soluble fibre within the diets (*e.g.* fruit and vegetables, especially legumes). Individuals participating in dietary intervention programs may be encouraged to consume equal amounts of polyunsaturated, mono-unsaturated and saturated fats by ensuring incorporation of olive oil (or equivalent) and sunflower oil (or equivalent) into each day's choices (in addition to saturated fat predominately from meat and dairy products). They may avoid using food products including specialist margarines which contain added plant sterols, omega-3 fatty acids or soy compounds, and soy based products. Furthermore, they may be encouraged to consume oily fish at least once a week within the fat restriction of the diet and they may attempt to maintain comparable ratios of simple sugars to complex carbohydrates. Individuals who are already taking vitamin and mineral supplementation before starting the dietary intervention program may continue taking the same dose throughout the program and this intake may be included in the intake analysis.

In an embodiment of the invention the marker is present in a locus, gene or gene cluster associated with an obesity-related phenotype. As used herein, "phenotype" refers to any observable or otherwise measurable physiological, morphological, biological, biochemical or clinical characteristic of an individual.

The SNP rs1900075 in additon to having a low p value and a high minor allele frequency has a significant interaction with a type of diet (*i.e.* the presence of this SNP is indicative that an individual is likely to lose more weight on a high fat/low carbohydrate diet than on a low fat/high carbohydrate diet). It is located within the EYA1 gene. The protein encoded by this gene may play a role in the developing kidney, branchial arches, eye, and ear. Mutations of this gene have been associated with branchiootorenal dysplasia syndrome, branchiootic syndrome, and sporadic cases of congenital cataracts and ocular anterior segment anomalies. A similar protein in mice can act as a transcriptional activator. This gene has not been previously shown to be involved in weight loss and/or weight management.

Of course, a combination of markers can be used in the methods, kits, uses, etc. of the present invention. Preferably, the markers are selected from SNP markers as set forth in Tables 1 and/or 2. Preferably, the markers are selected from the preferred SNP markers above. Markers may be present in coding (exons) but may also be present in non-coding regions (intron and intergenic regions). They may be present in different genes *e.g.* one marker in a first gene and another marker in a second gene. If more than one marker is used, the markers may be in linkage disequilibrium with one another, preferably in non-tight linkage disequilibrium. "Linkage disequilibrium" or "allelic association" means the preferential association of a particular allele or genetic marker with a specific allele or genetic marker at a nearby chromosomal location more frequently than expected by chance for any particular allele frequency in the population. Linkage disequilibrium may result from natural selection of certain combination of alleles or because an allele has been introduced into a population too recently to have reached equilibrium (random association) between linked alleles. A marker in linkage disequilibrium with disease predisposing variants can be particularly useful in detecting susceptibility to disease (or association with sub-clinical phenotypes), notwithstanding that the marker does not cause the phenotype. Methods to determine linkage disequilibrium are well known to the skilled artisan. The present invention thus also pertains to methods and uses comprising determining *in vitro* the genotype of SNP rs1900075 and/or at least one other SNP, *e.g.* another SNP presented in Table 1 and/or 2, in DNA taken from an individual. This other SNP may be in linkage disequilibrium with the first SNP.

Obesity-related phenotypes include, but are not limited to, body weight, BMI, percent fat mass, and serum triglycerides, cholesterol, and glucose, to name just a few. Genes associated with these phenotypes have been found (see Obesity: Genomics and postgenomics, Eds: Clement and Sorensen, Informa Healthcase, first edition, 2007). In a preferred embodiment the marker is rs1900075. It is to be understood that any marker that is in linkage disequilibrium with any of the SNPs shown in Table 1 and/or 2 can also be used in the various aspects and embodiments of the present invention. These markers do not necessarily have to be present in the same locus, gene or gene cluster as the markers shown in Table 1 and/or 2. They may be part of other more distant genes. However, they should be in linkage. "Linkage" describes the tendency of genes, alleles, loci or genetic markers to be inherited together as a result of their location on the same chromosome, and can be measured by percent recombination between the two genes, alleles, loci or genetic markers that are physically-linked on the same chromosome. Linkage disequilibrium can be determined in terms of r² which is the correlation coefficient and/or d which is the genetic distance. At least one of them should be above 0.8. Some linked markers occur within the same gene or gene cluster.

In a further aspect, the invention pertains to the use of rs1900075 for determining a diet an individual is most likely to complete successfully. In other words, the marker may be used for selecting an optimal diet for an individual. "Optimal" means, among others, that the individual should remain on the diet and complete it successfully *e.g.* should lose more weight on an optimal diet compared to a non-optimal diet. On the basis of a correlation, association, linkage or other relation between a genetic marker and the likelihood to remain on and successfully complete a specific diet, a suitable diet can be communicated, prescribed, suggested and/or recommended to an individual and/or added to an individual's food or diet. Preferably, the marker is a genetic marker such as a polymorphism, *e.g.* a SNP. From the genetic markers as shown herein marker rs1900075 can be selected the presence of which is indicative of an increased likelihood of an individual to successfully complete a high fat/low carbohydrate diet compared to a low fat/high carbohydrate diet. The invention therefore also pertains to a method for determining an optimal diet for an individual, the method comprising the steps of a) obtaining a biological sample comprising nucleic acid of the individual and b) genotyping the nucleic acid for SNP rs1900075 wherein the presence of this SNP is indicative that a high fat/low carbohydrate diet is the optimal diet for the individual.

As used herein "polymorphism" refers to DNA sequence variation in the cellular genome of an individual, typically with a population frequency of more than 1 %. A polymorphic marker or site is the locus at which genetic variation occurs. Preferred markers have at least two alleles, each occurring at a frequency of greater than 1 %, and more preferably greater than 10 % or 20 % of a selected population. A polymorphic locus may be as small as one base pair. Polymorphic markers include restriction fragment length polymorphisms, variable number of tandem repeats, hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wild-type form. Diploid organisms may be homozygous or heterozygous for allelic forms. A SNP occurs at a polymorphic site occupied by a single nucleotide. A SNP usually arises due to substitution of one nucleotide for another at the polymorphic site, but it can also arise from an insertion or deletion of a nucleotide relative to a reference allele.

The invention also pertains to a method for predicting the likelihood of success of an individual in a dietary weight loss intervention program, the method comprising the steps of obtaining a biological sample comprising nucleic acid of the individual and genotyping the nucleic acid for at least one single nucleotide polymorphism (SNP rs1900075), wherein the presence of rs1900075 is indicative of an increased likelihood of success of an individual in a dietary weight loss intervention program.

The individual may be overweight or obese. The dietary weight loss intervention program may comprise subjecting the individual to a hypo-caloric diet. In case an individual has the SNP rs1900075, it is preferred that the individual is subjected to a dietary weight loss intervention program comprising a high fat/low carbohydrate diet.

The present invention furthermore provides a method of determining whether an individual has an increased likelihood to successfully complete a specific diet, the method comprising the step of a) obtaining a biological sample comprising nucleic acid of the individual, and b) genotyping the nucleic acid for rs1900075. SNP rs1900075 associated with an increased likelihood of the individual to successfully complete a high fat/low carbohydrate diet. The SNPs can be used in the method of determining whether an individual has an increased predisposition to successfully complete a high fat/low carbohydrate diet or a low fat/high carbohydrate diet. The diets used in the methods and uses of the present invention are preferably hypo-energetic. The individuals may be overweight or obese.

The invention also pertains to a method for predicting whether an individual is likely to lose more weight on a high fat/low carbohydrate diet than on a low fat/high carbohydrate diet, the method comprising the steps of a) obtaining a biological sample comprising nucleic acid of the individual and b) genotyping the nucleic acid for at least the SNP rs1900075, wherein the presence of this SNP is indicative that an individual is likely to lose more weight on a high fat/low carbohydrate diet than on a low fat/high carbohydrate diet.

In the methods and uses of the present invention the occurrence of a specific allelic form (*e.g.* A allelic form) of a SNP may be assessed by contacting a nucleic acid derived from the genome of an individual with a first oligonucleotide that anneals with higher stringency with the specific allelic form (*e.g.* A allelic form) of the polymorphism than with another allelic form (*e.g.* T allelic form) of the polymorphism and assessing annealing of the first oligonucleotide and the nucleic acid, whereby annealing of the first oligonucleotide and the nucleic acid is an indication that the genome of the individual comprises the specific allelic form (*e.g.* A allelic form) of the polymorphism. The method may be extended by assessing the occurrence of the other allelic form (*e.g.* T allelic form) of the polymorphism by contacting the nucleic acid with a second oligonucleotide that anneals with higher stringency with the other allelic form (*e.g.* T allelic form) of the polymorphism than with the specific allelic form (*e.g.* A allelic form) of the polymorphism and assessing annealing of the second oligonucleotide and the nucleic acid, whereby annealing of the second oligonucleotide and the nucleic acid is an indication that at least one allele of the respective gene in the genome of the individual does not comprise the specific allelic form (*e.g.* A allelic form) of the polymorphism. The first and second oligonucleotides may be attached to a support. The support may be the same for both oligonucleotides.

"Biological sample" as used in the present invention encompasses a variety of sample types which can be used as source material for isolating nucleic acids. They include, but are not limited to, solid materials (*e.g.,* tissue, tissue cultures or cells derived there from and the progeny thereof, hair follicle samples, biopsy specimens, buccal cells provided by a swab, skin and nose samples) and biological fluids (*e.g.* urine, faecal material, blood, semen, amniotic fluid, tears, saliva, sputum, sweat, mouth wash). Any biological sample from a human individual comprising even one cell comprising nucleic acid can be used in the methods of the present invention. The term also includes samples that have been manipulated in any way after their procurement, such as by treatment with reagents, solubilisation, or enrichment for certain components, such as proteins or polynucleotides. The methods and uses of the present invention are preferably conducted on a sample that has previously been removed from the individual and do preferably not involve diagnosis practised on the human body.

Nucleic acid molecules as used herein refers to polymeric forms of nucleotides and includes both sense and antisense strands of RNA, cDNA, genomic DNA, and synthetic forms and mixed polymers of the above, with genomic DNA being preferred. A nucleotide refers to a ribonucleotide, deoxy(ribo)nucleotide or a modified form of either type of nucleotide. The term also includes single- and double-stranded forms of DNA. In addition, a polynucleotide may include either or both naturally-occurring and modified nucleotides linked together by naturally-occurring and/or non-naturally occurring nucleotide linkages. The nucleic acid molecules may be modified chemically or biochemically or may contain non-natural or derivatized nucleotide bases, as will be readily appreciated by those of skill in the art. Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. A reference to a nucleic acid sequence encompasses its complement unless otherwise specified. Thus, a reference to a nucleic acid molecule having a particular sequence should be understood to encompass its complementary strand, with its complementary sequence. The complementary strand is also useful, *e.g.,* for antisense therapy, hybridization probes and PCR primers.

Nucleic acids can be isolated from a particular biological sample using any of a number of procedures, which are well-known in the art, the particular isolation procedure chosen being appropriate for the particular biological sample. Methods of isolating and analyzing nucleic acid variants as described above are well known to one skilled in the art and can be found, for example in the Molecular Cloning: A Laboratory Manual, 3rd Ed., Sambrook and Russel, Cold Spring Harbor Laboratory Press, 2001 and Current Protocols in Molecular Biology Volumes I-III, 4th edition, Ausubel et al., John Wiley and Sons, 1995. Many of the methods require amplification of nucleic acid from target samples. This can be accomplished by techniques such as *e.g.* PCR, ligase chain reaction, nucleic acid based sequence amplification, self-sustained sequence replication and transcription amplification. Genetic markers such as the SNPs can be detected from the isolated nucleic acids using techniques including DNA hybridization methods (*e.g.* Southern Blotting, FISH), direct sequencing with radioactively, enzymatically, lumines-cently or fluorescently labelled primers (manually or automated), restriction fragment length polymorphism (RFLP) analysis, heteroduplex analysis, single strand conformational polymorphism (SSCP) analysis, denaturing gradient gel electrophoresis (DGGE), temperature gradient gel electrophoresis (TGGE), use of linked genetic markers, mass spectrometry *e.g.* MALDI-TOF, and chemical cleavage analysis to name just a few. Of course DNA MicroArray technology suitable for detecting genetic markers such as SNPs can also be used. All methods are explained in detail, for example, in the Molecular Cloning: A Laboratory Manual, 3rd Ed., Sambrook and Russel, Cold Spring Harbor Laboratory Press, 2001.

Primers used may be oligonucleotides hybridizing specifically with one allele. They are called allele-specific oligonucleotides. In the allele-specific PCR methodology, a target DNA is preferentially amplified only if it is completely complementary to the 3'-terminus of a specific PCR amplification primer. The 3'-terminus of the primer is designed so as to terminate at, or within one or two nucleotides of a known mutation site within the target DNA to which it possesses a complementary sequence. Under the appropriate reaction conditions, the target DNA is not amplified if there is a single nucleotide mismatch (*e.g.,* a nucleotide substitution caused by a mutation) or a small deletion or insertion, at the 3'-terminus of the primer. Accordingly, allele-specific PCR may be utilized to detect either the presence or absence of (at least) a single nucleotide mismatch between the primer sequence (which is complementary to a pre-selected target sequence) and a nucleic acid within the sample.

Amplification of the target sequence is indicative of a lack of even a single mismatched nucleotide. The markers in the present invention are preferably analyzed using methods amenable for automation. Primer extension analysis can be performed using any method known to one skilled in the art. Oligonucleotides, probes and/or primers may be naturally occurring or synthetic, but are typically prepared by synthetic means. They may be immobilized on a solid support. For instance, oligonucleotides, probes and/or primers as described herein can be used as a DNA chip. The chip may contain a primer corresponding to a single allelic form of a marker but may also contain a primer corresponding to both allelic forms of a marker. It may even comprise primers for different markers. The appropriate length of an oligonucleotide, probe and/or primer depends on its intended use but typically ranges from 10 to 75, preferably 15 to 40 nucleotides. Short primer molecules generally require cooler temperatures to form sufficiently stable hybrid complexes with the template. A primer need not reflect the exact sequence of the template but must be sufficiently complementary to hybridize with a template. Conditions suitable for hybridization are generally known in the art and will be apparent to the skilled artisan.

A non-limiting example of stringent hybridization conditions is hybridization in 6 x sodium chloride/sodium citrate (SSC) at about 45 °C, followed by one or more washes in 0.2 x SSC, 0.1 % SDS at 50 to 65 °C. Stringent conditions can for instance be found in Molecular Cloning: A Laboratory Manual, 3rd Ed., Sambrook and Russel, Cold Spring Harbor Laboratory Press, 2001 and Current Protocols in Molecular Biology, John Wiley & Sons, N.Y. (1989), 6.3.1-6.3.6. The term "primer site" refers to the area of the target DNA to which a primer hybridizes. The term "primer pair" means a set of primers including a 5'-upstream primer that hybridizes with the 5'-end of the DNA sequence to be amplified and a 3'-downstream primer that hybridizes with the complement of the 3'-end of the sequence to be amplified.

As used herein, "genotype" refers to the genetic constitution of an individual. More specifically, "genotyping" as used herein refers to the analysis of DNA in a sample obtained from a subject to determine the DNA sequence in a specific region of the genome, *e.g.* a locus that influences a trait. It may refer to the determination of DNA sequence at one or more polymorphic sites and/or determination of allelic patterns of an individual. The genotyping may be performed using a micro-array or a multi-well plate in for instance a laboratory or hospital. It may thus involve the use of a gene/DNA chip or a strip or solid surface comprising one or more nucleic acid molecules.

A further aspect of the invention pertains to a method for diagnosing an individual as being likely to succeed in a dietary weight loss intervention program, the method comprising the steps of genotyping nucleic acid of the individual for the SNP rs1900075. Furthermore, the invention is directed to a method for diagnosing an individual as being likely to succeed in a dietary weight loss intervention program, wherein the individual is treated with a specific type of diet *e.g.* a low fat/high carbohydrate diet or a high fat/low carbohydrate diet, the method comprising the steps of genotyping nucleic acid of the individual for SNP rs1900075.

The individual can be diagnosed as being likely to succeed in a dietary weight loss intervention program, wherein the individual is treated with a high fat/low carbohydrate diet, if the SNP marker rs1900075 is detected. The SNPs that are associated with successful completion of certain types of diet can be assessed from the results shown herein.

The invention also pertains to a method of assessing the desirability of treating an individual with a specific type of diet *e.g.* a hypo-caloric diet, a low fat/high carbohydrate diet or a high fat/low carbohydrate diet. In a further aspect the present invention provides a method of assessing the advisability that the individual should employ a dietary weight loss intervention program comprising a specific type of diet *e.g.* a hypo-caloric diet, a high fat/low carbohydrate diet or low fat/high carbohydrate diet. The invention further provides a method of assessing the desirability of supplementing the food of the individual with a specific type of diet *e.g.* a hypo-caloric diet, a high fat/low carbohydrate diet or low fat/high carbohydrate diet.

The invention is also directed to a method of determining whether an individual is a suitable candidate for a dietary weight loss intervention program comprising a specific type of diet *e.g.* a hypo-caloric diet, a high fat/low carbohydrate diet or low fat/high carbohydrate diet. In another aspect the invention relates to a method of assessing the advisability that an individual should employ a specific type of diet *e.g.* a hypo-caloric diet, a high fat/low carbohydrate diet or low fat/high carbohydrate diet. The above methods can be performed by identifying markers in nucleic acid of the individual that are indicative of an increased predisposition of the individual to successfully complete a dietary weight loss intervention program comprising a specific type of diet. The marker can be rs1900075. The above methods thus determine whether an individual such as an overweight or obese individual is or is not a suitable candidate for a weight management program comprising a specific dietary component. In the methods of the invention the high fat/low carbohydrate or low fat/high carbohydrate diet may be a hypo-energetic diet.

Furthermore, by using the markers found in the current invention the chance of losing weight with a diet, *e.g.* a hypo-energetic diet, can be indicated. Some people will lose weight easier than others and their genetic profile can indicate this. Based on this, nutritional and lifestyle advice with proper goal setting and management of expectations can be done by a health care professional which will lead to an increase in the chance of success for the individual.

Another aspect of the invention is directed to the use of a kit comprising at least one primer pair for genotyping the SNP rs1900075 and instructions explaining that detection of the presence of such SNP marker is indicative of a increased likelihood of success of an individual in a dietary weight loss intervention program in the method according to the present invention.

The invention thus also provides kits to determine whether an individual is resistant to weight loss based on analysis of genetic polymorphisms. The information can be used to screen individuals, *e.g.* overweight or obese individuals, and classify them based on their genetic tendency to lose weight or be resistant to lose weight. The polymorphisms as found herein are useful in predicting the outcome of bodyweight management strategies, particularly strategies having a component of dietary intervention either alone or as their main component. The kit comprises at least one primer or primer pair suitable for determining (or being associated with) the likelihood that an individual such as an overweight or obese individual successfully completes or unsuccessfully completes a dietary weight loss intervention program, more in particular a dietary component thereof such as a diet.

In an embodiment the invention is directed to a kit for use in a method or use according to the invention, the kit comprising at least one primer or primer pair for genotyping a marker in a gene or locus associated with obesity or an obesity-associated phenotype. Preferably, the marker is SNP rs1900075.

The primers may be suitable for nucleic acid sequence amplification. Often the kits contain one or more primers or primer pairs hybridizing to different forms of a polymorphism, *e.g.* a primer or primer pair capable of hybridizing to a first allelic form of a SNP (*e.g.* A allelic form) and a primer or primer pair capable of hybridizing to a second allelic form of the SNP (*e.g.* T allelic form).

Moreover, kits according to the invention may comprise instructions explaining correlation of the genotype to increased likelihood of successful completion of a specific type of diet such as a hypo-caloric diet, high fat/low carbohydrate diet or a low fat/high carbohydrate diet. Furthermore, the kit may comprise instructions explaining that detection of the presence and/or absence of certain SNPs, such as SNP rs1900075, is indicative of an increased predisposition of the individual to successfully complete a dietary weight loss intervention program comprising a specific type of diet, *e.g.* a low fat/high carbohydrate diet or a high fat/low carbohydrate diet. On the basis of the results obtained with the kit it can be detected if an individual has an increased likelihood to successfully complete a dietary weight loss intervention program comprising a high fat/low carbohydrate diet compared to a dietary weight loss intervention program comprising a low fat/high carbohydrate diet and *vice versa.* In an embodiment the invention provides a kit for use in a method according to the invention, the kit comprising at least one primer pair for genotyping the SNP rs1900075 and instructions explaining that detection of the presence of such SNP marker is indicative of a increased likelihood of success of an individual in a dietary weight loss intervention program.

In a further embodiment the invention provides the use of a kit comprising at least one primer pair for genotyping the SNP rs1900075 and instructions explaining that detection of the presence of the SNP rs1900075 is indicative that an individual is likely to lose more weight on a high fat/low carbohydrate diet than on a low fat/high carbohydrate in the method of the present invention.

Optional additional components of the kit include, for example, restriction enzymes, reverse transcriptase or polymerase, a positive control, a negative control, at least a further primer pair suitable for detecting (other) markers, appropriate buffers for reverse transcription, PCR and/or hybridization reactions, means used to label and nucleotide mix for the PCR reaction. The kits of the invention may thus also comprise one or more primers, primer pairs, probes and/or oligonucleotides suitable for detecting markers such as SNPs which is/are in linkage disequilibrium with a SNP as shown in Tables 1 and/or 2. It may also contain one or more primers, primer pairs, probes and/or oligonucleotides suitable for detecting another SNP as shown in one of the Tables 1 and/or 2.

In addition, a kit according to the present invention may contain instructions for carrying out the methods as well as a listing of the obesity-associated alleles and haplo-types relevant in view of the present invention. The components of the kit may be either in dry form in a tube or a vial or dissolved in an appropriate buffer.

The present invention employs, unless otherwise indicated, conventional (recombinant) techniques of molecular biology, immunology, microbiology, biochemistry and cell biology which are well within the skill of a person skilled in the art.

### Examples

To illustrate the invention, the following examples are provided. These examples are not intended to limit the scope of the invention.

### Example 1: Genome Wide Association Analysis

### Aim

A 10-week dietary weight loss intervention study was performed to examine the interaction between genetic factors and obesity related phenotypes. In order to achieve this goal, a whole genome association study was performed to identify genes associated with quantitative traits involved in weight loss/gain and in respect to co-variables of nutrient intake or more generally in respect to diet. Thus 318237 SNPs have been genotyped on the 771 obese individuals with the Illumina HumanHap 300-DUO SNP Chip.

### Description of the cohort

In a 10-week, European, multi-center dietary intervention study 771 weight stable, obese (BMI ≥ 30kg/m2), but otherwise healthy men and women were randomized to a low fat/high carbohydrate (20 to 25 % energy from fat; 60 to 65 % from carbohydrate) or high fat/low carbohydrate (40 to 45 % energy from fat; 40 to 45 % from carbohydrate), hypo-energetic diet (energy deficiency of 600 kcal/day).

### Selection of patients

Obese subjects were recruited from May 2001 until September 2002. Inclusion criteria were: BMI ≥ 30 and age 20 to 50. Exclusion criteria were: weight change > 3 kg within the last 3 months prior to study start, hypertension, diabetes or hyperlipidemia treated by drugs, untreated thyroid disease, surgically or drug-treated obesity, pregnancy, and participation in other trials, and alcohol or drug abuse. Informed written consent was obtained prior to study participation and the study was approved by the Ethical Committee at each of the participating centers. The study has been described in detail elsewhere (see Petersen *et al.* (2006) and Sorensen *et al.* (2006)).

### Analysis of phenotypes

Body weights were measured on calibrated scales. Waist circumferences were measured with the participant wearing only non-restrictive underwear. Body height was measured with a calibrated stadiometer. The mean of three measurements was recorded for each variable. Fat mass and fat-free mass were assessed by multifrequency bio-impedance (Bodystat; QuadScan 4000, Isle of Man, British Isles). Resting metabolic rate was measured by ventilated hood systems routinely used at each centre and a standardized validation program was used to facilitate pooling of the results from the different centres. Venous blood samples were drawn after an overnight fast of 12 hours, following a 3-day period when subjects had been instructed to avoid excessive physical activity or alcohol consumption. Subjects rested in the supine position for 15 minutes prior to the procedure. Insulin secretion and insulin resistance were measured by HOMA.Statistical modelling. Separate linear regression models were made for effect on weight loss, change in fasting glucose, change in fasting insulin, change in insulin secretion and change in insulin resistance.

### Covariates

Statistical analyses were adjusted for baseline weight at the beginning of the intervention (as described in Sorensen *et al.* (2006)), gender, age, center and diet group.

### Preparation of samples

High molecular weight genomic and mitochondrial DNA was isolated from blood samples using routine methods. Concentration of purified DNA in each sample was measured using Syber Green II quantification method. For genotyping using the Illumina platform a minimum of 750 ng with a concentration of 50 ng/µl of genomic DNA is necessary, therefore each DNA sample was diluted accordingly. Of the 771 samples from obese individuals, 751 met these criteria.

### Genome-wide Scanning Using Illumina HumanHap 300-DUO Chips

The whole genome genotyping of the DNA samples was performed using the Illumina HumanHap 300-DUO SNP BeadChips and Infinium II genotyping assay. The HumanHap 300-DUO BeadChips contains over 317000 SNP markers of which the majority are tagSNP markers derived from the International HapMap Project. TagSNPs are loci that can serve as proxies for many other SNPs. The use of tagSNPs greatly improves the power of association studies as only a subset of loci needs to be genotyped while maintaining the same information and power as if one had genotyped a larger number of SNPs.

The Infinium II genotyping with the HumanHap300DUO BeadChips were performed according to the "Single-Sample BeadChip Manual process" described in detail in "Infinium™ II Assay System Manual" provided by Illumina (San Diego, CA, USA). Briefly, 750 ng of genomic DNA from a sample was subjected to whole genome amplification. The amplified DNA was fragmented, precipitated and resuspended in hybridization buffer. The resuspended sample was heat denatured and then applied to one HumanHap300DUO BeadChip. After overnight hybridization, mis- and non-hybridized DNA was washed away from the BeadChip and allele-specific single-base extension of the oligonucleotides on the BeadChip was performed, using labelled deoxynucleotides and the captured DNA as a template. After staining of the extended DNA, the BeadChips were washed and scanned with the BeadArray Reader (Illumina) and genotypes from samples were called by using the BeadStudio software (Illumina). All 751 DNA samples that met the quality requirements of the Illumina platform were genotyped.

### Statistical Analysis of the GWS data of the Nugenob Study:

Firstly, quality control measures were done. All genotyped SNPs were tested for Hardy Weinberg equilibrium using a package R:genetics. SNPs which showed a deviation from the Hardy Weinberg equilibrium were flagged to investigate any possible genotyping errors.

To study the population structure a random set of 27974 SNPs covering all auto-somal chromosomes were selected and then analysed using the plink software that uses complete linkage agglomerative clustering, based on pair wise identity-by-state distance. In addition, 602 ancestry informative markers for European populations were selected to detect population stratification using plink and STRUCTURE software. The conclusion of all analyses was that there was no significant population stratification in this study cohort.

Call frequencies (number of delivered genotypes per SNP) for more than 99 % of the SNPs were ≥ 98 %. Call rates (number of genotyped SNPs per individual) for more than 98% of the individuals were ≥ 95 %, both consistent with the specifications of the manufacturer, indicating an accurate and reliable genotyping.

Then, statistical analysis was done. Concerning the association between weight loss and genetic component, multiple linear regression analysis using HelixTree (Gold-enHelix Inc.) using gender, age, center, diet group and weight at the beginning of the intervention as covariates and several models (additive, dominant and recessive) well known to the person skilled in the art using a multiple linear regression with R statistical software controlling for gender, age, center, diet group, and weight at the beginning of the intervention, with and without controlling for an interaction between diet and genetic component, as described in *e.g.* Sorensen *et al.* (2006) were applied.

Genome wide scanning - whole genome association analysis in weight loss using European, multi-center dietary intervention study subjects and Illumina HumanHap-300DUO BeadChips was carried out. The final data set used in the statistical analysis included 750 subjects. In this study new weight loss associated SNP markers, of which several are intragenic, were found.

### Example 2: Integrative Analysis

Scientific literature was carefully mined for genes that previously have been associated with dietary weight loss or fat oxidation capacity. Based on the InWeb protein-protein interaction database (see Lage *et al.,* 2007), all proteins in this database were ranked according to their proximity to these candidate proteins in the protein-protein interaction network.

Next, GWAS-based weight loss evidence (with and without diet interaction) and GWAS-based fat oxidation evidence layers were constructed by ranking all genes-products according to the significance of their harboring SNPs. Each GWAS-based evidence layer was collapsed with the protein-protein interaction rank to yield three metaranks. Each meta-rank reflects the ranking of all proteins according to their degree of interaction with previously reported weight loss genes or fat oxidation genes and their significance in the GWAS.

### Example 3: Fine Mapping

For genes that encompass the SNPs with the lowest p values from the genome wide association analysis (example 1) and/or the integrative analysis (example 2), all the SNPs within these genes were selected for fine mapping. In addition, for the SNPs with the lowest p values from the genome wide association analysis (example 1) and/or the integrative analysis (example 2) that are not located within a gene, other SNPs in their vicinity were selected for fine mapping. This resulted in 1536 SNPs. The geno-types of these 1536 SNPs were queried using custom-made Illumina GoldenGate genotyping assay according to the instructions of the manufacturer. Briefly, 250 ng of DNA was activated and assay oligonucleotides, hybridization buffer, and paramagnetic particles were combined with the activated DNA. After hybridization and several wash steps, allele-specific oligonucleotides were extended and ligated to locus-specific oligonucleotides. The resulting products were then amplified and labeled using PCR, hybridized to the array matrix and visualized using the Illumina Bead Array Reader. This resulted in new weight loss associated SNP markers. The new SNP markers are shown in Tables 1 and 2.

In Table 1 the SNPs that are associated with weight loss without considering a diet-gene interaction are shown. In Table 2 the SNP markers associated with weight loss considering a low fat/high carbohydrate diet - gene interaction or a high fat/low carbohydrate diet - gene interaction are shown. The beta-value for the SNPs of Table 2 was calculated by introducing a diet - gene interaction term in the regression formula. The direction and the magnitude of the beta value shows the direction and the magnitude of the diet - gene interaction, *e.g.* if an individual has an SNP with a negative beta value the individual will lose more weight on a low fat/high carbohydrate diet than on a high fat/low carbohydrate diet and if an individual has an SNP with a positive beta value the individual will lose more weight on a high fat/low carbohydrate diet than on a low fat/high carbohydrate diet. In Table 3 the SNP markers associated with weight loss considering a high fat/low carbohydrate diet - gene interaction are shown, while in Table 4 the SNP markers associated with weight loss considering a low fat/high carbohydrate diet - gene interaction are shown. In Table 5 the sequence of SNPrs1900075 is shown.

### References

Lage K, Karlberg EO, Størling ZM, Olason PI, Pedersen AG, Rigina O, Hinsby AM, Tümer Z, Pociot F, Tommerup N, Moreau Y and Brunak S (2007), A human phenomeinteractome network of protein complexes implicated in genetic disorders. Nat. Biotechnol. 25: 309-316.
Petersen M, Taylor MA, Saris WHM, Verdich C, Toubro S, Macdonald I, Rossner S, Stich V, Guy-Brand B, Langin D, Martinez JA, Pedersen O, Holst C, Sorensen TIA, Astrup A and The Nugenob Consortium (2006), Randomized, multi-center trial of two hypo-energetic diets in obese subjects: high- versus low-fat content. Int. J. Obes. (Lond.). 30: 552-60.
Sorensen TIA, Boutin P, Tayloer MA, Larsen LH, Verdich C, Petersen L, Holst C, Echwald SM, Dina C, Tourbo S, Petersen M, Polak J, Clement K, Martinez JA, Langin D, Oppert J_M, Stch V, Macdonald I, Amer P, Saris WHM, Pedersen O, Astrup A, Froguel P and The Nugenob Consortium (2006), Genetic polymorphisms and weight loss in obesity: a randomised trial of hypo-energetic high- versus low-fat diets. PLoS Clinical Trials 1(2):e12.

**Table 1: List of SNPs associated with weight loss without considering a diet-gene interaction.**

| rs number | Chrom osome | Position¹ | MAF² | p-value³ | Analysis Model⁴ | Gene name⁵ |
|---|---|---|---|---|---|---|
| rs560514 | 1 | 18005.187 | 48.6 | 0.0077258 | dom | ACTL8 |
| rs1402694 | 1 | 84373.101 | 47 | 0.0029393 | rec | PRKACB |
| rs1402696 | 1 | 84495.181 | 27.9 | 0.0032752 | add | PRKACB |
| rs1402695 | 1 | 84495.257 | 32.3 | 0.0037994 | add | PRKACB |
| rs486708 | 1 | 179726.011 | 30.3 | 0.0045287 | dom | CACNA1E |
| rs943795 | 1 | 179729.548 | 30.8 | 0.0040956 | rec | CACNA1E |
| rs1574781 | 1 | 179801.093 | 16.7 | 0.0002271 | rec | CACNA1E |
| rs 199939 | 1 | 179862.601 | 17 | 0.0037988 | add | CACNA1E |
| rs6429280 | 1 | 239793.335 | 6.7 | 0.0028134 | dom | KMO |
| rs632172 | 1 | 239813.048 | 24 | 0.0015949 | add | KMO;OPN3 |
| rs850678 | 1 | 239813.762 | 24.1 | 0.0016709 | add | KMO;OPN3 |
| rs659887 | 1 | 239814.626 | 24 | 0.0018518 | add | KMO;OPN3 |
| rs7582990 | 2 | 10450.319 | 46.3 | 0.0020863 | dom | HPCAL1 |
| rs6432096 | 2 | 10478.833 | 47.2 | 0.0060517 | dom | HPCAL1; AX748389; ODC1 |
| rs1974676 | 2 | 10481.363 | 48.4 | 0.0050683 | add | HPCAL1; AX748389; ODC1 |
| rs3755259 | 2 | 10483.483 | 30 | 0.0020384 | dom | HPCAL1; AX748389; ODC1 |
| rs3755256 | 2 | 10483.865 | 24.9 | 0.001783 | rec | HPCAL1; AX748389; ODC1 |
| rs6734108 | 2 | 104911.319 | 27.4 | 0.0067197 | dom | - |
| rs6735232 | 2 | 105038.671 | 22.1 | 0.000623 | add | MRPS9 |
| rs4848123 | 2 | 121298.58 | 10.5 | 0.0071225 | dom | GLI2;hGli2 |
| rs10173252 | 2 | 121365.713 | 12.6 | 0.0003926 | add | GLI2;hGli2 |
| rs13382915 | 2 | 121366.367 | 11.9 | 0.0051576 | dom | GLI2;hGli2 |
| rs6434276 | 2 | 188871.362 | 31.2 | 0.000177 | add | GULP1;CED-6 |
| rs12693496 | 2 | 188895.64 | 31.4 | 0.0002358 | add | GULP1;CED-6 |
| rs12105671 | 2 | 188901.387 | 20.1 | 0.0060611 | add | GULP1;CED-6 |
| rs4274570 | 2 | 188975.067 | 17.8 | 0.0072836 | rec | GULP1;CED-6 |
| rs6723034 | 2 | 189023.584 | 18.7 | 0.0094718 | dom | GULP1;CED-6 |
| rs2043448 | 2 | 203754.77 | 42.4 | 0.0002238 | add | NBEAL1 |
| rs12693982 | 2 | 203793.88 | 38.8 | 0.0088351 | add | NBEAL1; ALS2CR17; CYP20A1 |
| rs 1376877 | 2 | 203980.335 | 43.9 | 0.000199 | add | ABI2;argBPIB |
| rs2469954 | 2 | 204000.762 | 39.6 | 0.0008401 | add | ABI2;argBPIB; RAPH1 |
| rs2250522 | 2 | 204009.541 | 39.3 | 0.0002388 | rec | ABI2;argBPIB; RAPH1 |
| rs2246849 | 2 | 204017.57 | 40.4 | 0.001115 | add | ABI2;argBPIB; RAPH1 |
| rs11687186 | 2 | 204028.084 | 43.7 | 0.0003165 | add | RAPH1 |
| rs2246118 | 2 | 204035.884 | 39.8 | 0.0015805 | dom | RAPH1 |
| rs2469962 | 2 | 204064.067 | 37 | 0.0027434 | rec | RAPH1 |
| rs6436943 | 2 | 231065.616 | 15.9 | 0.0003553 | add | SP100 |
| rs836230 | 2 | 231067.342 | 22.3 | 0.000149 | rec | SP100 |
| rs836235 | 2 | 231110.472 | 22.5 | 0.0030107 | dom | SP100 |
| rs6728423 | 2 | 231114.773 | 16.6 | 0.0036066 | add | SP100 |
| rs6729378 | 2 | 231118.764 | 16.2 | 0.001526 | add | SP100 |
| rs4683301 | 3 | 46906.482 | 39.8 | 0.009668 | rec | PTHR1 |
| rs1138518 | 3 | 46919.277 | 37.1 | 0.0036716 | dom | PTHR1; CCDC12 |
| rs7652849 | 3 | 46923.529 | 10.3 | 0.0036529 | rec | PTHR1; CCDC12 |
| rs9855938 | 3 | 46932.301 | 35 | 0.0002074 | add | PTHR1; CCDC12 |
| rs9825199 | 3 | 196385.872 | 7 | 0.0015826 | add | C3orf21 |
| rs3796160 | 3 | 196387.902 | 6.8 | 0.0008579 | add | C3orf21 |
| rs9870813 | 3 | 196405.418 | 40.1 | 0.0047172 | add | C3orf21 |
| rs823504 | 3 | 196766.921 | 43.6 | 0.0002504 | dom | PPP1R2; APOD |
| rs10512926 | 5 | 7548.654 | 11.9 | 0.0061115 | rec | ADCY2 |
| rs7701465 | 5 | 75472.144 | 42.8 | 0.0088649 | dom | SV2C |
| rs13179555 | 5 | 75483.95 | 13.7 | 0.008348 | rec | SV2C; RAP1B |
| rs6887093 | 5 | 75491.007 | 5 | 0.0024241 | add | SV2C; RAP1B |
| rs2358531 | 5 | 75515.541 | 27.1 | 0.006301 | dom | SV2C; RAP1B |
| rs1002541 | 5 | 75518.657 | 40.6 | 0.0003338 | add | SV2C; RAP1B |
| rs12153396 | 5 | 75521.611 | 13.2 | 0.0041712 | dom | SV2C; RAP1B |
| rs884948 | 5 | 75530.933 | 21.9 | 6.16e-05 | rec | SV2C |
| rs4704296 | 5 | 75538.166 | 46.7 | 0.0078475 | rec | SV2C |
| rs4704297 | 5 | 75541.953 | 46.2 | 0.004968 | rec | SV2C |
| rs2937723 | 5 | 75596.304 | 49.1 | 0.0040707 | rec | SV2C |
| rs2937719 | 5 | 75600.192 | 47.8 | 0.0032118 | dom | SV2C |
| rs203138 | 6 | 138640.977 | 30.9 | 0.0082285 | add | KIAA1244 |
| rs203133 | 6 | 138716.446 | 30.8 | 6.32e-05 | dom | KIAA1244 |
| rs6965716 | 7 | 5300.735 | 46.6 | 0.0013148 | rec | SLC29A4; KIAA1856; TNRC18 |
| rs2685753 | 7 | 5304.623 | 24.5 | 0.0014555 | rec | SLC29A4; KIAA1856; TNRC18 |
| rs3889348 | 7 | 5305 | 35.4 | 0.0087289 | dom | SLC29A4; KIAA1856; TNRC18 |
| rs1230544 | 7 | 52760.864 | 48.8 | 0.0052908 | dom | - |
| rs10488501 | 7 | 52897.21 | 7.3 | 0.0088172 | rec | - |
| rs3779340 | 7 | 77506.111 | 7.3 | 0.0008011 | add | MAGI2 |
| rs10486838 | 7 | 77510.477 | 7.7 | 0.0010904 | add | MAGI2 |
| rs10486839 | 7 | 77510.796 | 7.7 | 0.0012399 | add | MAGI2 |
| rs11763565 | 7 | 77510.931 | 7.6 | 0.0013407 | add | MAGI2 |
| rs3807778 | 7 | 77511.416 | 7.7 | 0.0010489 | add | MAGI2 |
| rs10277160 | 7 | 77513.065 | 48 | 0.0037096 | add | MAGI2 |
| rs11768469 | 7 | 77514.331 | 7.7 | 0.0010489 | add | MAGI2 |
| rs3779331 | 7 | 77515.078 | 17.4 | 0.0012737 | dom | MAGI2 |
| rs1031177 | 8 | 72359.211 | 49.4 | 0.0007133 | add | EYA1 |
| rs8181006 | 8 | 72360.941 | 49.7 | 0.0089052 | add | EYA1 |
| rs7822041 | 8 | 72375.246 | 49.9 | 0.0092251 | add | EYA1 |
| rs10092844 | 8 | 72375.843 | 40.4 | 0.0049002 | add | EYA1 |
| rs1900075 | 8 | 72380.735 | 24.7 | 7.1e-05 | rec | EYA1 |
| rs2120995 | 8 | 72384.023 | 44.3 | 0.0039051 | add | EYA1 |
| rs4295694 | 8 | 72388.952 | 44.3 | 0.0057616 | add | EYA1 |
| rs10104134 | 8 | 72401.188 | 38.7 | 0.0052419 | dom | EYA1 |
| rs8176747 | 9 | 135121.136 | 9.2 | 0.0067107 | rec | ABO |
| rs568203 | 9 | 135141.265 | 22.7 | 0.008145 | add | ABO |
| rs651007 | 9 | 135143.696 | 23.6 | 0.0033268 | add | ABO |
| rs579459 | 9 | 135143.989 | 23.6 | 0.0034911 | add | ABO |
| rs635634 | 9 | 135144.821 | 21 | 0.0009335 | add | ABO |
| rs633862 | 9 | 135145.265 | 45 | 0.0019862 | add | ABO |
| rs558240 | 9 | 135146.954 | 40 | 0.0008559 | rec | ABO |
| rs487820 | 9 | 135202.19 | 46.6 | 0.007621 | add | SURF6; SURF5;RPL7A SURF1;SURF2 SURF4 |
| rs2051680 | 9 | 135212.61 | 9 | 0.0037903 | dom | SURF6; SURF5;RPL7A SURF1;SURF2 SURF4 |
| rs1179037 | 9 | 135228.33 | 46.8 | 0.0078819 | add | SURF1; SURF2;SURF4 C9orf96 |
| rs3739892 | 9 | 135228.74 | 8.3 | 0.0035985 | rec | SURF1; SURF2;SURF4 C9orf96 |
| rs3758348 | 9 | 135229.22 | 16 | 0.0001599 | add | SURF1; SURF2;SURF4 C9orf96 |
| rs4623810 | 10 | 121151.787 | 38.7 | 0.0077544 | dom | GRK5 |
| rs 10886489 | 10 | 121213.977 | 33.1 | 0.003126 | rec | GRK5 |
| rs2991769 | 10 | 121221.429 | 29.4 | 0.0014087 | add | GRK5 |
| rs2104992 | 10 | 121222.72 | 38.4 | 0.0039575 | rec | GRK5 |
| rs12766539 | 10 | 121223.24 | 14.2 | 0.0040419 | add | GRK5 |
| rs2991770 | 10 | 121224.052 | 29.8 | 0.0001632 | add | GRK5 |
| rs7081349 | 10 | 128778.701 | 13.5 | 0.0070685 | dom | DOCK1 |
| rs11016125 | 10 | 128779.08 | 13.9 | 0.0069815 | rec | DOCK1 |
| rs6482668 | 10 | 128792.938 | 13.8 | 0.0067912 | rec | DOCK1 |
| rs11016240 | 10 | 128802.674 | 13.2 | 0.009395 | rec | DOCK1 |
| rs1761534 | 10 | 128811.186 | 13.2 | 0.009795 | dom | FLJ45557; DOCK1 |
| rs2255615 | 10 | 128812.817 | 13.2 | 0.0099225 | dom | FLJ45557; DOCK1 |
| rs2791754 | 10 | 128817.695 | 13.3 | 0.009795 | dom | FLJ45557; DOCK1 |
| rs928571 | 10 | 128895.709 | 31.9 | 1.24e-05 | rec | FLJ45557; DOCK1 |
| rs731644 | 11 | 76073.936 | 31.7 | 0.0092649 | dom | LRRC32; AB231718 |
| rs 10899257 | 11 | 76092.856 | 13.8 | 0.0002621 | add | AB231718 |
| rs10793186 | 11 | 76104.841 | 5.4 | 0.0074861 | rec | AB231718 |
| rs9568494 | 13 | 50460.372 | 24.6 | 0.009331 | add | RNASEH2B; GUCY1B2 |
| rs7337462 | 13 | 50523.974 | 31.9 | 0.005993 | rec | GUCY1B2 |
| rs6561608 | 13 | 50535.096 | 30.6 | 0.0052615 | rec | GUCY1B2 |
| rs 16945369 | 15 | 89561.829 | 11.5 | 0.0042657 | rec | SV2B |
| rs6496772 | 15 | 89562.558 | 11.6 | 0.0058606 | dom | SV2B |
| rs6496774 | 15 | 89571.651 | 20.2 | 0.0073788 | add | SV2B |
| rs11865234 | 16 | 1136.76 | 20.6 | 0.0009341 | add | CACNA1H |
| rs909910 | 16 | 1138.938 | 20 | 0.0003899 | add | CACNA1H |
| rs8047814 | 16 | 73793.654 | 34.5 | 3.22e-05 | rec | CTRB2;CTRB1 |
| rs11149808 | 16 | 73811.261 | 48.4 | 0.0037215 | dom | CTRB2;CTRB1; BCAR1 |
| rs12443712 | 16 | 73848.113 | 30.7 | 0.0028398 | rec | BCAR1 |
| rs7230580 | 18 | 3626.125 | 34 | 0.0050308 | rec | DLGAP1 |
| rs7359820 | 18 | 3713.036 | 8.8 | 0.0094112 | rec | DLGAP1 |
| rs1433840 | 18 | 36255.129 | 17.9 | 0.0058643 | rec | - |
| rs226313 | 18 | 36599.186 | 22.4 | 0.0065655 | dom | - |
| rs2123473 | 18 | 36873.597 | 7.2 | 0.0099958 | add | - |
| rs8088748 | 18 | 38120.51 | 10.5 | 0.0019241 | add | LOC284260 |
| rs10502781 | 18 | 38134.527 | 9.2 | 0.0012676 | dom | LOC284260 |
| rs9946713 | 18 | 38144.54 | 28.6 | 0.0049178 | add | LOC284260 |
| rs7504768 | 18 | 38861.218 | 42.4 | 0.0082532 | add | RIT2 |
| rs8098098 | 18 | 42164.419 | 20.6 | 0.0076017 | add | C18orf23;RNF 165 |
| rs7242055 | 18 | 42209.698 | 10 | 0.0001293 | rec | RNF165 |
| rs1470324 | 18 | 42212.17 | 36.2 | 0.009492 | rec | RNF165 |
| rs4890647 | 18 | 42215.275 | 8.7 | 8.14e-05 | rec | RNF165 |
| rs9974676 | 21 | 25926.919 | 23.8 | 0.0024126 | dom | JAM2 |
| rs974680 | 21 | 25932.982 | 15.1 | 0.001207 | add | JAM2 |
| rs4816260 | 21 | 25935.229 | 24.7 | 0.0011907 | add | JAM2 |
| rs11087969 | 21 | 25941.197 | 24.7 | 0.0016657 | add | JAM2 |
| rs2829850 | 21 | 25951.611 | 25.9 | 0.0031597 | add | JAM2 |
| rs7283477 | 21 | 25987.023 | 20.5 | 0.0030217 | dom | JAM2 |
| rs2829875 | 21 | 26006.867 | 15.9 | 0.0035462 | add | JAM2;ATP5J |
| rs135570 | 22 | 44911.444 | 49.2 | 0.0008589 | dom | PPARA |
| rs135549 | 22 | 44931.971 | 43.8 | 0.0047657 | dom | PPARA |

| | | | | | | |
|---|---|---|---|---|---|---|
| ¹ means position on chromosome ² means minor allele frequency ³ means the statistical significance with the model used ⁴ the model used for the statistical analysis (dominant, recessive or additive) ⁵ means the gene(s) in the vicinity of the SNP, genes determined as the -20 kb of the start codon and +20 kb of the stop codon of a gene | | | | | | |

**Table 2: SNP markers associated with weight loss considering a low fat/high carbohydrate diet - gene interaction or a high fat/low carbohydrate diet - gene interaction.**

| rs number | Chrom osome | Position¹ | Beta² | p-value³ | MAF⁴ | gene name⁵ |
|---|---|---|---|---|---|---|
| rs12125019 | 1 | 179737.471 | 5.6222832 | 0.0017432 | 12.5 | CACNA1E |
| rs 17494681 | 1 | 179746.805 | - 1.3024102 | 0.0159862 | 18.5 | CACNA1E |
| rs6743846 | 2 | 10397.865 | - 1.1311254 | 0.0274621 | 14 | HPCAL1 |
| rs 10495589 | 2 | 12124.827 | 1.3019828 | 0.0157476 | 41.6 | AK001558 |
| rs266065 | 2 | 103434.175 | 0.7780197 | 0.0358498 | 46.3 | - |
| rs2672847 | 2 | 103437.967 | 1.0594337 | 0.0041554 | 48.1 | - |
| rs1992902 | 2 | 121387.072 | - 1.3982458 | 0.0082493 | 14.1 | GLI2;hGIi2 |
| rs11687797 | 2 | 188848.297 | 1.3494852 | 0.0008309 | 29.2 | GULP1;CED-6 |
| rs11687248 | 2 | 188852.866 | 0.8171732 | 0.0263272 | 40 | GULP1;CED-6 |
| rs6434274 | 2 | 188868.949 | 0.9075492 | 0.0206976 | 31.4 | GULP1;CED-6 |
| rs4233800 | 2 | 188889.84 | 0.8917972 | 0.0227298 | 31.4 | GULP1;CED-6 |
| rs12105671 | 2 | 188901.387 | 1.1496286 | 0.0110662 | 20.1 | GULP1;CED-6 |
| rs1900075 | 8 | 72380.735 | 1.2562617 | 0.0163825 | 24.7 | EYA1 |
| rs7036324 | 9 | 135106.579 | - 1.7620918 | 0.0044782 | 10.6 | ABO |
| rs7914808 | 10 | 120991.172 | -6.281477 | 0.0049618 | 11.5 | GRK5 |
| rs11854719 | 15 | 89618.817 | 2.5618139 | 0.0011751 | 34 | SV2B |
| rs12599288 | 16 | 11729.566 | - 1.0612499 | 0.039891 | 27.8 | TXNDC11 |
| rs 1559361 | 16 | 73787.515 | 5.0079337 | 0.0011499 | 15.9 | CTRB2 |
| rs7238810 | 18 | 37716.012 | 1.4485406 | 0.000147 | 42.2 | - |
| rs4630636 | 18 | 37996.502 | -1.37969 | 0.0098752 | 21.4 | - |
| rs9956391 | 18 | 38031.27 | 1.2608104 | 0.0048229 | 21.1 | LOC284260 |
| rs4816260 | 21 | 25935.229 | 1.1115316 | 0.0310329 | 24.7 | JAM2 |
| rs11087969 | 21 | 25941.197 | 1.0770327 | 0.038203 | 24.7 | JAM2 |
| rs2829843 | 21 | 25945.544 | 0.9118593 | 0.0455153 | 20.2 | JAM2 |
| rs2829850 | 21 | 25951.611 | - 1.1176838 | 0.0308793 | 25.9 | JAM2 |
| rs2829875 | 21 | 26006.867 | -1.224864 | 0.0338886 | 15.9 | JAM2;ATP5J |

| | | | | | | |
|---|---|---|---|---|---|---|
| ¹ means position on chromosome ² means beta value for the gene-diet interaction term ³ means the statistical significance of the interaction ⁴ means minor allele frequency ⁵ means the gene(s) in the vicinity of the SNP, genes determined as the -20 kb of the start codon and +20 kb of the stop codon of a gene | | | | | | |

**Table 3: SNP markers associated with weight loss considering a high fat/low carbohydrate diet - gene interaction.**

| rs number | Chrom osome | Position¹ | Beta² | p-value³ | MAF⁴ | gene name⁵ |
|---|---|---|---|---|---|---|
| rs12125019 | 1 | 179737.471 | 5.6222832 | 0.0017432 | 12.5 | CACNA1E |
| rs10495589 | 2 | 12124.827 | 1.3019828 | 0.0157476 | 41.6 | AK001558 |
| rs266065 | 2 | 103434.175 | 0.7780197 | 0.0358498 | 46.3 | - |
| rs11687797 | 2 | 188848.297 | 1.3494852 | 0.0008309 | 29.2 | GULP1;CED-6 |
| rs11687248 | 2 | 188852.866 | 0.8171732 | 0.0263272 | 40 | GULP1;CED-6 |
| rs6434274 | 2 | 188868.949 | 0.9075492 | 0.0206976 | 31.4 | GULP1;CED-6 |
| rs4233800 | 2 | 188889.84 | 0.8917972 | 0.0227298 | 31.4 | GULP1;CED-6 |
| rs12105671 | 2 | 188901.387 | 1.1496286 | 0.0110662 | 20.1 | GULP1;CED-6 |
| rs1900075 | 8 | 72380.735 | 1.2562617 | 0.0163825 | 24.7 | EYA1 |
| rs11854719 | 15 | 89618.817 | 2.5618139 | 0.0011751 | 34 | SV2B |
| rs1559361 | 16 | 73787.515 | 5.0079337 | 0.0011499 | 15.9 | CTRB2 |
| rs9956391 | 18 | 38031.27 | 1.2608104 | 0.0048229 | 21.1 | LOC284260 |
| rs4816260 | 21 | 25935.229 | 1.1115316 | 0.0310329 | 24.7 | JAM2 |
| rs11087969 | 21 | 25941.197 | 1.0770327 | 0.038203 | 24.7 | JAM2 |
| rs2829843 | 21 | 25945.544 | 0.9118593 | 0.0455153 | 20.2 | JAM2 |

| | | | | | | |
|---|---|---|---|---|---|---|
| ¹ means position on chromosome ² means beta value for the gene-diet interaction term ³ means the statistical significance of the interaction ⁴ means minor allele frequency ⁵ means the gene(s) in the vicinity of the SNP, genes determined as the -20 kb of the start codon and +20 kb of the stop codon of a gene | | | | | | |

**Table 4: SNP markers associated with weight loss considering a low fat/high carbohydrate diet - gene interaction.**

| rs number | Chro mosome | Position¹ | Beta² | p-value³ | MAF⁴ | gene name⁵ |
|---|---|---|---|---|---|---|
| rs17494681 | 1 | 179746.805 | -1.3024102 | 0.0159862 | 18.5 | CACNA1E |
| rs6743846 | 2 | 10397.865 | -1.1311254 | 0.0274621 | 14 | HPCAL1 |
| rs2672847 | 2 | 103437.967 | -1.0594337 | 0.0041554 | 48.1 | - |
| rs1992902 | 2 | 121387.072 | -1.3982458 | 0.0082493 | 14.1 | GLI2;hGli2 |
| rs7036324 | 9 | 135106.579 | -1.7620918 | 0.0044782 | 10.6 | ABO |
| rs7914808 | 10 | 120991.172 | -6.281477 | 0.0049618 | 11.5 | GRK5 |
| rs12599288 | 16 | 11729.566 | -1.0612499 | 0.039891 | 27.8 | TXNDC11 |
| rs7238810 | 18 | 37716.012 | -1.4485406 | 0.000147 | 42.2 | - |
| rs4630636 | 18 | 37996.502 | -1.37969 | 0.0098752 | 21.4 | - |
| rs2829850 | 21 | 25951.611 | -1.1176838 | 0.0308793 | 25.9 | JAM2 |
| rs2829875 | 21 | 26006.867 | -1.224864 | 0.0338886 | 15.9 | JAM2;ATP5 J |

| | | | | | | |
|---|---|---|---|---|---|---|
| ¹ means position on chromosome ² means beta value for the gene-diet interaction term ³ means the statistical significance of the interaction ⁴ means minor allele frequency ⁵ means the gene(s) in the vicinity of the SNP, genes determined as the -20 kb of the start codon and +20 kb of the stop codon of a gene | | | | | | |

**Table 5: SNP marker rs1900075 including its nucleotide sequence.**

| rs number | Nucleotide sequence |
|---|---|
| rs1900075 | TTGCATCACAACAACCTCTAGAGTTG[C/T]ACCTATAAAGAATGCCAGTAG ATGC (SEQ ID NO. 1) |

## Claims

1. Use of a single nucleotide polymorphism (SNP), rs1900075, for predicting the likelihood of success of an individual in a dietary weight loss intervention program comprising subjecting the individual to a hypo-caloric diet.

2. Use according to claim 1, **characterized in that** the individual is overweight or obese.

3. A method for predicting the likelihood of success of an individual in a dietary weight loss intervention program, the method comprising the steps of:
a) obtaining a biological sample comprising nucleic acid of the individual,
b) genotyping the nucleic acid for at least one SNP according to claim 1, wherein the presence of the SNP claim 1 is indicative of an increased likelihood of success of an individual in a dietary weight loss intervention program.

4. A method for predicting whether an individual is likely to lose more weight on a high fat/low carbohydrate diet than on a low fat/high carbohydrate diet, the method comprising the steps of:
a) obtaining a biological sample comprising nucleic acid of the individual,
b) genotyping the nucleic acid for at least one SNP, rs1900075, wherein the presence of this SNP is indicative that an individual is likely to lose more weight on a high fat/low carbohydrate diet than on a low fat/high carbohydrate diet.

5. Use of a kit comprising at least one primer pair for genotyping the SNP, rs1900075, and instructions explaining that detection of the presence of such SNP marker is indicative of a increased likelihood of success of an individual in a dietary weight loss intervention program in the method of claim 3.

6. Use of a kit comprising at least one primer pair for genotyping the SNP rs1900075, and instructions explaining that detection of the presence of such a SNP marker is indicative that an individual is likely to lose more weight on a high fat/low carbohydrate diet than on a low fat/high carbohydrate diet in the method of claim 4.

7. Use of a kit according to claim 5 or 6, further comprising at least one component selected from the group consisting of a restriction enzyme, a reverse transcriptase or polymerase, a positive control, a negative control, at least a further primer pair suitable for detecting (other) markers, an appropriate buffer for reverse transcription, a PCR and/or a hybridization reaction, a means used to label and a nucleotide mix for the PCR reaction in the method of claim 4.

## Patentansprüche

1. Verwendung eines Einzelnukleotidpolymorphismus (SNP), rs1900075, zum Prognostizieren der Erfolgswahrscheinlichkeit von einem Individuum bei einem diätassoziierten Gewichtsverlustinterventionsprogramm, bei dem man das Individuum einer kalorienarmen Diät unterwirft.

2. Verwendung nach Anspruch 1, **dadurch gekennzeichnet, dass** das Individuum übergewichtig oder fettleibig ist.

3. Verfahren zum Prognostizieren der Erfolgswahrscheinlichkeit eines Individuums bei einem diätassoziierten Gewichtverlustinterventionsprogramm, wobei das Verfahren die folgenden Schritte umfasst:
a) Gewinnen einer nukleinsäurehaltigen biologischen Probe des Individuums,
b) Genotypisieren der Nukleinsäure auf mindestens einen SNP nach Anspruch 1, wobei das Vorhandensein von SNP nach Anspruch 1 eine erhöhte Erfolgswahrscheinlichkeit eines Individuums bei einem diätassoziierten Gewichtsverlustinterventionsprogramm anzeigt.

4. Verfahren zum Prognostizieren, ob bei einem Individuum die Wahrscheinlichkeit besteht, dass es mehr Gewicht mit einer stark fetthaltigen und kohlenhydratarmen Diät verliert als mit einer fettarmen und stark kohlenhydrathaltigen Diät, wobei das Verfahren die folgenden Schritte umfasst:
a) Gewinnen einer nukleinsäurehaltigen biologischen Probe des Individuums,
b) Genotypisieren der Nukleinsäure auf mindestens einen SNP, rs1900075, wobei das Vorhandensein dieses SNP anzeigt, dass bei einem Individuum die Wahrscheinlichkeit besteht, dass es mehr Gewicht mit einer stark fetthaltigen und kohlenhydratarmen Diät als mit einer fettarmen und stark kohlenhydrathaltigen Diät verliert.

5. Verwendung eines Kits, umfassend mindestens ein Primerpaar zum Genotypisieren des SNP, rs1900075, und Anweisungen, aus denen hervorgeht, dass der Nachweis des Vorhandenseins von solch einem SNP-Marker ein Zeichen dafür ist, dass bei einem Individuum eine erhöhte Erfolgswahrscheinlichkeit mit einem diätassoziierten Gewichtsverlustinterventionsprogramm in dem Verfahren nach Anspruch 3 besteht.

6. Verwendung eines Kits, umfassend mindestens ein Primerpaar zum Genotypisieren des SNP, rs1900075, und Anweisungen, aus denen hervorgeht, dass der Nachweis des Vorhandenseins von solch einem SNP-Marker ein Zeichen dafür ist, dass bei einem Individuum die Wahrscheinlichkeit besteht, dass es mehr Gewicht mit einer stark fetthaltigen und kohlenhydratarmen Diät als bei einer fettarmen und stark kohlenhydrathaltigen Diät in dem Verfahren nach Anspruch 4 verliert.

7. Verwendung eines Kits nach Anspruch 5 oder 6, weiter umfassend mindestens eine Komponente, ausgewählt aus der Gruppe bestehend aus einem Restriktionsenzym, einer reversen Transkriptase oder Polymerase, einer Positivkontrolle, einer Negativkontrolle, mindestens einem weiteren Primerpaar, das geeignet ist, um (andere) Marker nachzuweisen, einem entsprechenden Puffer für die reverse Transkription, einer PCR-und/oder einer Hybridisierungsreaktion, einem Mittel zum Markieren und einem Nukleotid-Cocktail für die PCR-Reaktion in dem Verfahren nach Anspruch 4.

## Revendications

1. Utilisation d'un polymorphisme d'un seul nucléotide (SNP), rs1900075, pour prédire la probabilité du succès d'un individu dans un programme d'intervention de perte de poids alimentaire comprenant la soumission de l'individu à un régime hypocalorique.

2. Utilisation selon la revendication 1, **caractérisée en ce que** l'individu est en surpoids ou obèse.

3. Procédé pour prédire la probabilité du succès d'un individu dans un programme d'intervention de perte de poids alimentaire, le procédé comprenant les étapes de :
a) obtention d'un échantillon biologique comprenant un acide nucléique de l'individu,
b) génotypage de l'acide nucléique pour au moins un SNP selon la revendication 1, où la présence du SNP de la revendication 1 est indicatif d'une probabilité accrue de succès d'un individu dans un programme d'intervention de perte de poids alimentaire.

4. Procédé pour prédire si un individu est susceptible de perdre plus de poids avec un régime riche en lipides/pauvre en glucides qu'avec un régime pauvre en lipides/riche en glucides, le procédé comprenant les étapes de :
a) obtention d'un échantillon biologique comprenant un acide nucléique de l'individu,
b) génotypage de l'acide nucléique pour au moins un SNP, rs1900075, où la présence de ce SNP est une indication qu'un individu est susceptible de perdre plus de poids avec un régime riche en lipides/pauvre en glucides qu'avec un régime pauvre en lipides/riche en glucides.

5. Utilisation d'un kit comprenant au moins une paire d'amorces pour génotyper le SNP, rs1900075, et des instructions expliquant que la détection de la présence d'un tel marqueur SNP est indicative d'une probabilité de succès augmentée d'un individu dans un programme d'intervention de perte de poids alimentaire dans le procédé de la revendication 3.

6. Utilisation d'un kit comprenant au moins une paire d'amorces pour génotyper le SNP rs1900075, et des instructions expliquant que la détection de la présence d'un tel marqueur SNP est une indication qu'un individu est susceptible de perdre plus de poids avec un régime riche en lipides/pauvre en glucides qu'avec un régime pauvre en lipides/riche en glucides dans le procédé de la revendication 4.

7. Utilisation d'un kit selon la revendication 5 ou 6, comprenant en outre au moins un composant choisi dans le groupe constitué d'un enzyme de restriction, une transcriptase inverse ou polymérase, un témoin positif, un témoin négatif, au moins une autre paire d'amorces adaptée pour détecter des (autres) marqueurs, un tampon approprié pour la transcription inverse, une réaction de PCR et/ou d'hybridation, un moyen utilisé pour marquer et un mélange de nucléotides pour la réaction de PCR dans le procédé de la revendication 4.
